Europäisches Patentamt

European Patent Office

Office européen des brevets

(19)

(11) Veröffentlichungsnummer: **0 015 486**
**B1**

(12) **EUROPÄISCHE PATENTSCHRIFT**

(45) Veröffentlichungstag der Patentschrift:
04.01.84

(51) · Int. Cl.³: **C 07 C 79/37**, C 07 C 76/02

(21) Anmeldenummer: **80100950.7**

(22) Anmeldetag: **26.02.80**

(54) **Verfahren zur Herstellung von 4,5-Dinitro-1,8-dihydroxyanthrachinon.**

(30) Priorität: **10.03.79 DE 2909481**

(43) Veröffentlichungstag der Anmeldung:
**17.09.80 Patentblatt 80/19**

(45) Bekanntmachung des Hinweises auf die Patenterteilung:
**04.01.84 Patentblatt 84/1**

(84) Benannte Vertragsstaaten:
**CH DE FR GB**

(56) Entgegenhaltungen:
**EP - A - 0 000 341**
**DE - A - 2 810 484**

*Die Akte enthält technische Angaben, die nach dem Eingang der Anmeldung eingereicht wurden und die nicht in dieser Patentschrift enthalten sind.*

(73) Patentinhaber: **BAYER AG, Zentralbereich Patente, Marken und Lizenzen, D-5090 Leverkusen 1, Bayerwerk (DE)**

(72) Erfinder: **Steinbeck, Werner, Dr., Wolfskaul 8, D-5000 Köln 80 (DE)**
Erfinder: **Gehrke, Günter, Dr., Leopold-Gmelin-Strasse 26, D-5000 Köln 80 (DE)**

Verfahren zur Herstellung von 4,5-Dinitro-1,8-dihydroxyanthrachinon

Verfahren zur Herstellung von 4,5-Dinitro-1,8-dihydroxy-anthrachinon

Gegenstand der Erfindung ist ein verbessertes Verfahren zur Herstellung von technisch reinem 4,5-Dinitro-1,8-dihydroxyanthrachinon. Dieses wichtige Farbstoffzwischenprodukt wurde bislang in technisch reiner Form auf die Weise hergestellt, dass man Anthrachinon-1,8-disulfonsäure mit Natriumchlorat in 1,8-Dichloranthrachinon überführt und dieses mit Natriumphenolat zum 1,8-Diphenoxy-anthrachinon umsetzt, welches dann nitriert und schliesslich verseift wird.

Wegen der Vielstufigkeit dieses Verfahrens und der Notwendigkeit zur Beseitigung des bei der Hydrolyse des Nitrierungsproduktes zwangsläufig anfallenden Dinitrophenols ist dieses Verfahren jedoch verhältnismässig teuer. Weniger aufwendig und damit bedeutend wirtschaftlicher ist die Herstellung von 4,5-Dinitro-1,8-dihydroxyanthrachinon, wenn man Anthrachinon-1,8-disulfonsäure mit Calciumhydroxyd in 1,8-disulfonsäure mit Calciumhydoxyd in 1,8-Dihydroxyanthrachinon umwandelt und dieses mit Salpetersäure bei Gegenwart von Borsäure nitriert oder aber die genannte Disulfonsäure mittels methanolischer Kalilauge in 1,8-Dimethoxyanthrachinon überführt, dieses anschliessend in Schwefelsäure nitriert und die Methoxygruppen verseift (vgl. z.B. Endeavour XXXV, Seite 137, Sept. 1976).

Beide Varianten dieser «Direktnitrierung» weisen jedoch den Nachteil auf, dass dabei als unerwünschte Nebenprodukte grössere Mengen des 2,5-Dinitroderivates sowie Trinitroverbindungen entstehen.

Es wurde nun gefunden, dass man ein weitgehend von Nebenprodukten freies, im allgemeinen 95-99 %iges 4,5-Dinitro-1,8-dihydroxyanthrachinon durch «Direktnitrierung» von 1,8-Dihydroxyanthrachinon bzw. 1,8-Dimethoxyanthrachinon erhält, wenn man die Säurekonzentration so wählt, dass nach Abschluss der Nitrierungs- und gegebenenfalls Verseifungsreaktionen eine 80-100 %ige Schwefelsäure (bezogen auf den Wassergehalt) vorliegt, bzw. durch Zugabe von Wasser eine derartige Schwefelsäurekonzentration eingestellt wird, und das auskristallisierende 4,5-Dinitro-1,8-dihydroxyanthrachinon abtrennt.

Das Abtrennen des gewünschten Reaktionsproduktes erfolgt in üblicher Weise durch Filtration und anschliessendes Waschen mit Schwefelsäure und Wasser, wobei die unerwünschten Nebenprodukte praktisch vollständig in Lösung bleiben und gewünschtenfalls durch Ausfällen mit Wasser aus der Mutterlauge isoliert werden können.

Es muss als ausgesprochen überraschend angesehen werden, dass nach dem erfindungsgemässen Verfahren auf einfache Weise ein hochreines Verfahrensprodukt erhalten wird, nachdem aus der DE-A 28 10 484 (Seite 5, Absatz 2) und aus der EP-A 00 00 341 (Seite 1 unten) bekannt war, dass man reine Produkte nicht durch direkte Nitrierung des entsprechenden Dihydroxyanthrachinons, sondern nur über den Umweg der Phenylether gewinnen kann.

Bei der praktischen Durchführung des beanspruchten Verfahrens geht man im allgemeinen so vor, dass man 1 Teil 1,8-Dihydroxyanthrachinon in einer Lösung von 0,25 - 0,75 Teilen Borsäure in 4 - 20 Teilen konzentrierter Schwefelsäure oder Oleum löst und mit 0,50 - 0,55 Teilen Salpetersäure oder der entsprechenden Menge Mischsäure in an sich bekannter Weise -10°C bis 30°C nitriert. Dabei wird die gesamte Salpetersäuremenge verbraucht. Danach wird durch vorsichtige Zugabe von Wasser die geforderte Schwefelsäurekonzentration von 30 - 100%, vorzugsweise 85 - 90% eingestellt. Vorteilhafterweise wird dabei die Verdünnungswärme nicht sofort durch äussere Kühlung abgeführt, um ein langsames Auskristallisieren des gewünschten Produktes zu ermöglichen. Bei der anderen Variante des erfindungsgemässen Verfahrens wird 1 Teil 1,8-Dimethoxyanthrachinon in 4 - 20 Teilen 90 - 100 %iger Schwefelsäure mit 0,45 - 0,50 Teilen Salpetersäure oder der entsprechenden Menge Mischsäure ohne Zusatz von Borsäure bei -10°C bis 30°C nitriert. Anschliessend werden durch mehrstündiges Erhitzen auf 90 - 120°C die Methoxygruppen verseift.

Bei beiden Varianten lässt man das Reaktionsgemisch langsam bis auf Raumtemperatur erkalten, bevor man 4,5-Dinitro-1,8-dihydroxyanthrachinon scharf absaugt und mit Schwefelsäure und anschliessend mit Wasser wäscht.

4,5-Dinitro-1,8-dihydroxyanthrachinon ist, wie bereits erwähnt, ein bedeutendes Zwischenprodukt zur Herstellung wertvoller Farbstoffe. Beispielsweise erhält man daraus durch Reduktion der Nitrogruppen und ggf. anschliessende Halogenierung klare blaue Polyesterfarbstoffe (vgl. z.B. US-PS 2 990 413). Durch Austausch einer Nitrogruppe gegen Arylaminreste und ggf. anschliessende Reduktion der verbliebenen Nitrogruppe erhält man ebenfalls wertvolle Polyesterfarbstoffe (vgl. US-PS 2 053 274)

Die Umsetzung von 4,5-Dinitro-1,8-dihydroxyanthrachinon mit ggf. substituiertem Anilin bei Gegenwart von Borsäure liefert 1,4-Dianilino-5-nitro-8-hydroxyanthrachinone, grüne Polyesterfarbstoffe (vgl. z.B. US-PS 3 444 215).

Durch Reduktion einer Nitrogrupppe im 4,5 Dinitro-1,8-dihydroxyanthrachinon und anschliessende Umsetzung mit Alkyl- oder Arylaminen bei Gegenwart von Borsäure erhält man 1-Alkyl(Aryl)amino-4-amino-5-nitro-8-hydroxyanthrachinone, türkisblaue Polyesterfarbstoffe (vgl. US-PS 3 883 567).

Das nach dem erfindungsgemässen Verfahren erhältliche 4,5-Dinitro-1,8-dihydroxyanthrachinon ist von hoher Qualität und kann direkt ohne weitere Reinigungsoperationen für derartige Farbstoffsynthesen eingesetzt werden.

Im Gegensatz dazu liefern die nach dem Stand der Technik erhaltene «Direktnitrierungsprodukte» qualitativ minderwertige Farbstoffe mit trübem Farbton.

Bei den oben genannten und in den nachstehenden Beispielen erwähnten «Teilen» handelt es sich um Gewichtsteile.

Beispiel 1

In einer Lösung von 32 Teilen Borsäure in 760 Teilen 20 %igem Oleum werden bei maximal 50°C 64 Teile 90 %iges 1,8-Dihidroxy-anthrachinon gelöst. Dann lässt man unter Kühlen bei 0 - 5°C innerhalb von 3 Stunden 103 Teile Mischsäure (33% $HNO_3$, 67% $H_2SO_4$) zutropfen und rührt noch 1 Stunde bei dieser Temperatur weiter. In 1,5 Stunden werden nun 133 Teile Wasser zugetropft, wobei die Temperatur bis 100°C ansteigen darf. Man rührt 1 Stunde bei 100°C, kühlt auf 20 - 25°C herunter und saugt die kristalline Fällung ab. Man wäscht mit 145 Teilen 88 %iger Schwefelsäure und darauf mit Wasser und erhält nach dem Trocknen 59,5 Teile 99,2 %iges 4,5-Dinitro-1,8-dihydroxy-anthrachinon, welches 0,5% 2,5-Dinitroverbindung und 0,2% 2,4,5-Trinitroderivat enthält.

Aus der Mutterlauge und der Schwefelsäure lassen sich durch Austragen auf Eiswasser, Filtrieren, Neutralwaschen mit Wasser und Trocknen 22 Teile Feststoff isolieren. Darin wurde durch quantitative Säulenchromatographie folgende Nitrierungsprodukte bestimmt:

21,2% 4,5 Dinitro- DHA,
43,7% 2,5-Dinitro- DHA,
  2,7% 2,7- Dinitro- + 2,4,7-Trinitro- DHA,
  5,7% 2,4,5-Trinitro-DHA,
  1,4% 4-Nitro- NHA.
(1,8-Dihydroxy-anthrachinon = DHA)

Beispiel 2

Man verfährt wie im Beispiel 1 angegeben. Nach der Nitrierung tropft man jedoch 155 Teile Wasser statt 133 Teile zu. Man erhält 66 Teile 96,9 %iges 4,5-Dinitro-1,8-dihydroxy-anthrachinon, das 0,9% 2,5-Dinitroisomeres und eine Spur Trinitroderivat enthält.

Aus der Mutterlauge und der Schwefelsäure gewinnt man 17 Teile Feststoff folgender Zusammensetzung:

  7,5% 4,5-Dinitro- DHA,
53,9% 2,5-Dinitro- DHA,
  2,7% 2,7-Dinitro- + 2,4,7-Trinitro- DHA,
  1,6% 4-Nitro- DHA.

Beispiel 3

Die Arbeitsweise ist die gleiche wie im Beispiel 1, jedoch wird in 320 Teilen 20 %igem Oleum statt in 760 Teilen nitriert und anschliessend nur mit 38 Teilen Wasser verdünnt. Ausbeute 54 Teile 95,1 %iges 4,5-Dinitro-1,8-dihydroxy-anthrachinon mit 2,8% 2,5-Dinitro-und 0,5% Trinitroverbindung.

Aus der Mutterlauge und der Schwefelsäure erhält man 28 Teile Feststoff mit folgenden Analysenwerten:

20,3% 4,5-Dinitro-DHA,
51,9% 2,5-Dinitro-DHA,
  3,9% 2,7-Dinitro- +2,4,7,-Trinitro-DHA,
  1,0% 2,4,5-Trinitro-DHA,
  1,4% 4-Nitro-DHA.

Beispiel 4

64 Teile 89,5 %iges 1,8-Dimethoxy-antrachinon werden bei 20°C in 740 Teilen 96 %iger Schwefelsäure gelöst. Zu dieser Lösung tropft man unter Kühlen im Verlauf von 3 Stunden bei 0 - 5°C 92 Teile Mischsäure (33% $HNO_3$, 67% $H_2SO_4$) und rührt 1 Stunde nach. Man heizt in 1 Stunde auf 100 - 105°C, hält diese Temperatur 4 Stunden lang, kühlt danach auf 20 - 25°C, saugt ab und wäscht mit 180 Teilen 96 %iger Schwefelsäure und anschliessend mit Wasser. Nach dem Trocknen erhält man 64 Teile 98,7 %iges 4,5-dihydroxy-anthrachinon, das 1% 2,5-Dinitroderivat enthält.

Behandelt man die Mutterlauge und die Schwefelsäurewäsche wie im Beispiel 1 beschrieben, so erhält man 10 Teile Nebenprodukte.

Die Beispiele 1 - 4 können auch in der Weise modifiziert werden, dass man den gesamten Nitrierungsansatz auf Eis austrägt und den gesamten Niederschlag mit 80 - 100 %iger Schwefelsäure behandelt, so dass die Nebenprodukte in Lösung gehen. Diese Verfahrensweise bietet jedoch technisch keine Vorteile gegenüber den oben beschriebenen Methoden.

**Patentansprüche**

1. Verfahren zur Herstellung von 4,5-Dinitro-1,8-dihydroxy-anthrachinon durch Nitrierung von 1,8-Dihydroxy-anthrachinon in gegebenenfalls $SO_3$-haltiger Schwefelsäure bei Gegenwart von Borsäure bzw. durch Nitrierung von 1,8-Dimethoxyanthrachinon in Schwefelsäure und anschliessende Verseifung der Methoxygruppen, dadurch gekennzeichnet, dass man die Säurekonzentration so wählt, dass nach Abschluss der Nitrierungs- und gegebenenfalls Verseifungsreaktion eine 80 - 100 %ige Schwefelsäure - bezogen auf den Wassergehalt - vorliegt bzw. durch Zugabe von Wasser sich eine derartige Schwefelsäurekonzentration einstellt und dass auskristallisierende 4,5-Dinitro-1,8-dihydroxy-anthrachinon abtrennt.

2. Verfahren gemäss Anspruch 1, dadurch gekennzeichnet, dass man nach der Nitrierung des 1,8-Dihydroxy-anthrachinons durch Zugabe von Wasser eine 85 - 90 %ige Schwefelsäure einstellt.

3. Verfahren nach Anspruch 1, dadurch gekennzeichnet, dass man die Säurekonzentration bei der Nitrierung und Verseifung von 1,8-Dimethoxy-anthrachinon so wählt, dass sich nach beendeter Reaktion unmittelbar eine 90 - 99 %ige Schwefelsäurekonzentration einstellt.

**Claims**

Process for the preparation of 4,5-dinitro-1,8-dihydroxy-anthraquinone by nitration of 1,8-dihydroxy-anthraquinone in sulphuric acid, which optionally contains $SO_3$, in the presence of boric acid or by nitration of 1,8-dimethoxyanthraquinone in sulphuric acid and subsequent saponification of the methoxy groups, characterised in that the acid concentrations are chosen such that, when the nitration reaction and if appropriate the saponification reaction have ended, an 80 - 100% strength sulphuric acid - based on the contend of water - is present, or such a sulphuric acid concentration is established by adding water, and the 4,5-dinitro-1,8-dihydroxy-anthraquinone which crystallises out is separated off.

2. Process according to Claim 1, characterised in that, after nitration of the 1,8-dihydroxy-anthraquinone, an 85 - 90% strength sulphuric acid is established by adding water.

3. Process according to Claim 1, characterised in that the acid concentration in the nitration and saponification of 1,8-dimethoxy-anthraquinone is chosen such that, when the reaction has ended, a sulphuric acid concentration of 90 - 99% is established immediately.

**Revendications**

1. Procédé de préparation de la 4,5-dinitro-1,8-dihydroxy-anthraquinone par nitration de la 1,8-dihydroxyanthraquinone dans de l'acide sulfurique contenant éventuellement $SO_3$ en présence d'acide borique ou, respectivement, par nitration de la 1,8-diméthoxy-anthraquinone dans l'acide sulfurique avec saponification subséquente des groupes méthoxy, caractérisé en ce que l'on choisit la concentration de l'acide de manière qu'après la réaction de nitration et éventuellement la réaction de saponification on obtienne un acide sulfurique à 80 - 100% (pour la teneur en humidité), ou bien, respectivement, on règle à cette concentration en acide par addition d'eau et on sépare la 4,5-dinitro-1,8-dihydroxyanthraquinone qui cristallise.

2. Procédé selon la revendication 1, caractérisé en ce que, après la nitration de la 1,8-dihydroxyanthraquinone, on règle l'acide sulfurique à 85 - 90% par addition d'eau.

3. Procédé selon la revendication 1, caractérisé en ce que, à la nitration et à la saponification de la 1,8-diméthoxyanthraquinone, on choisit la concentration en acide de manière qu'après la réaction il s'établisse directement une concentration en acide sulfurique de 90 à 99%.